Europäisches Patentamt

(19) European Patent Office

Office européen des brevets

(11) Publication number: **0 320 879**
**A1**

(12) # EUROPEAN PATENT APPLICATION

(21) Application number: 88120814.4

(22) Date of filing: 13.12.88

(51) Int. Cl.4: **C07C 85/26 , C07C 87/62 , C07C 87/58 , B01D 3/36**

(30) Priority: 14.12.87 US 132491

(43) Date of publication of application:
21.06.89 Bulletin 89/25

(84) Designated Contracting States:
BE CH DE LI

(71) Applicant: **AIR PRODUCTS AND CHEMICALS, INC.**
**Route no. 222**
**Trexlertown Pennsylvania 18087(US)**

(72) Inventor: **Fowlkes, Robert Lee**
**220 Cedar Street**
**Milton Florida 32570(US)**

(74) Representative: **Dipl.-Ing. Schwabe, Dr. Dr. Sandmair, Dr. Marx**
**Stuntzstrasse 16**
**D-8000 München 80(DE)**

(54) Separating toluenediamine from N-tertiarybutyl toluenediamine.

(57) This invention relates to an improved process for separating an azeotrope of toluenediamine from N-tertiary-butyl-toluenediamine by contacting the azeotrope with a hydrocarbon which forms a lower boiling azeotrope with toluenediamine and one in which the solubility of toluenediamine is less than 30 grams/100 grams of hydrocarbon.

EP 0 320 879 A1

## SEPARATING TOLUENEDIAMINE FROM N-TERTIARYBUTYL TOLUENEDIAMINE

### BACKGROUND OF THE INVENTION

Alkylated and N-alkyl aromatic amines are known and have been used as antioxidant additives for enhancing the resistance or organic materials to oxidation and as intermediates for industrial chemicals. Alkylated aromatic amines and N-alkylated aromatic amines have been prepared by a variety of techniques which typically involve the reaction of an alcohol with an aromatic amine in the presence of a catalyst or by the reaction of an olefin with an aromatic amine. Another technique is to react an amine hydrohalide with an alcohol to produce N-alkylated products. Representative patents which show the preparation of ring alkylated and N-alkylated aromatic amines are as follows:

U.S. 2,692,287 and U.S. 2,692,288 disclose a process for producing N-tertiary-alkylated aromatic amines by reacting a tertiary alcohol such as tertiary butanol with an aromatic amine. Representative systems include the production of N-tertiary-butyl aniline, N-tertiary-butyl-p-nitroaniline, N-tertiary-butyl-2,napthylamine and N-tertiary-butyl-2,4-diaminotoluene.

European patents disclose a process for producing tertiary-butyl toluenediamines by the reaction of isobutylene with toluenediamine in the presence of an acidic zeolite catalyst, silica-alumina, or an acid catalyst such as hydrochloric acid.

In the reaction to produce tertiary-butyl-toluenediamine as represented by the above patents, a reaction product containing toluenediamine and N-tertiary-butyl-toluenediamine and ring alkylated toluenediamine is produced. In many instances, it is desirable to recover the N-tertiary-butyl-toluenediamine from the reaction mixture. This is particularly true in the case where the N-tertiary-butyl-toluenediamine is to be used as an antioxidant material. However, in those instances where separation is required, separation is difficult because toluenediamine forms an azeotrope with N-tertiary-butyl-toluenediamine which cannot be separated by distillation. Thus, straight-forward distillation is not possible, and as represented in U.S. 2,692,287, a multi-step extraction process with two-step crystallization has been utilized.

### SUMMARY OF THE INVENTION

This invention relates to an improved process for separating, an azeotrope of toluenediamine from N-tertiary-butyl-toluenediamine. The improvement for enhancing the separation of toluenediamine from N-tertiary-butyl-toluenediamine and for breaking the toluenediamine-N-tertiary-butyl-toluenediamine azeotrope comprises contacting the azeotrope with a $C_{14}$-$C_{16}$ hydrocarbon having a boiling point ranging from about 240-290°C atmospheric pressure which forms a lower boiling azeotrope with toluenediamine and not with N-tertiary-butyl-toluenediamine and a hydrocarbon in which the toluenediamine is not soluble in an amount exceeding about 30 grams per 100 grams hydrocarbon.

### DETAILED DESCRIPTION OF THE INVENTION

The feedstock suited for treatment by this process is an azeotropic feedstock containing toluenediamine and N-tertiary-butyl-toluenediamine. The azeotrope may be generated by a number of processes. Examples of processes suited for producing this feedstock include the reaction of isobutanol with toluenediamine, the reaction of isobutylene with toluenediamine, and the reaction of the hydrohalide of toluenediamine with tertiary-butyl alcohol.

The hydrocarbon which is to be used in breaking the azeotrope of toluenediamine and N-tertiary-butyl-toluenediamine is a hydrocarbon having from about 14 to 16 carbon atoms and one that has a boiling point ranging from about 240-290°C at atmospheric pressure. Further, the hydrocarbon is one which forms an azeotrope with toluenediamine which has a lower boiling point, i.e., at least 10°C preferably 20°C lower than the boiling point of the azeotrope of toluenediamine and N-tertiary-butyl-toluenediamine (176°C at 25 mmHg). Further, the hydrocarbon should be one in which toluenediamine is not soluble to an extent greater than 30 grams preferably 12 grams per 100 grams of hydrocarbon. Examples of hydrocarbons meeting these requirements are tetradecane, tetradecene and hexadecane. Aromatic hydrocarbons, xylene, dodecane and decalin are examples of hydrocarbons not meeting these criteria because toluenediamine is either too soluble for permitting ease of separation from the solvent or does not form a lower boiling azeotrope.

In the overall process of breaking the azeotrope of toluenediamine and N-tertiary-butyl-toluenediamine, the azeotrope is contacted with the hydrocarbon typically in amount to provide from about 50 to 500 preferably 75-150 parts by weight hydrocarbon per 100 parts of azeotrope. Distillation of the mixture consisting of the azeotrope of the hydrocarbon and toluenediamine and N-tertiary-butyl-toluenediamine is then effected in conventional manner. An overhead fraction of the azeotrope of hydrocarbon and toluenediamine is removed as an overhead in the distillation process and N-tertiary-butyl-toluenediamine is removed as a bottoms fraction. Because of the limited solubility of toluenediamine in the hydrocarbon solvent, toluenediamine then can be removed from the azeotrope by decanting.

The following examples are provided to illustrate various embodiments of the invention.

Example 1

Conventional Distillation

A 2,000 gram portion of crude product having approximately 28.5% toluenediamine (TDA), 11.1% N-tertiary-butyl-toluenediamine (NTBTDA), 52.7% tertiary-butyl-toluenediamine (TBTDA) and 7.7% of other components from the reaction of isobutylene and toluenediamine was charged to a still and distilled at a pressure of 25 mm Hg. The reflux ratio was 10.1. With 30% of the charge taken as overhead the highest concentration of toluenediamine in the overhead was 80% by weight which resulted in a concentration of about 20% of N-tertiary-butyl-toluenediamine in the bottoms fraction. The azeotropic boiling point at this pressure was approximately 176°C. With 10% removal of the initial charge as overhead, the highest concentration of N-tertiary-butyl-toluenediamine in the bottoms fraction was 37% and a 50% concentration of toluenediamine in the overheads. Neither embodiment, i.e., the 10% or 30% overhead removal shows effective separation of toluenediamine from N-t-butyltoluenediamine.

Example 2

The process of Example 1 was repeated except that 200 grams of reactor product of similar composition to that used in Example 1 and 200 grams of various hydrocarbons were charged to the pot of the distillation still to determine their efficiency in enhancing separation. Table 1 sets forth a listing of the hydrocarbons, pressure, boiling point of azeotrope, the percent of the hydrocarbon in the azeotropic composition with toluenediamine and the percent of toluenediamine in the upper and lower layers of the decanter.

TABLE 1

| HYDROCARBONS USED TO REMOVE TDA FROM TDA - NTBTDA AZEOTROPE | | | | | | |
|---|---|---|---|---|---|---|
| Hydrocarbon | Pressure mm Hg | Azeotrope bp T°C | Wt% Azeotropic Composition | | Decanter Upper Level % TDA | Decanter Lower Level % HC |
| | | | HC | TDA | | |
| Tetradecane | 25 | 136 | 89 | 11 | 5.2 | 0.8 |
| | 50 | 153 | 89 | 11 | | |
| | 100 | 195 | 89 | 11 | | |
| Hexadecane | 25 | 142 | 76 | 24 | | 0.7 |
| | 50 | 174 | 74 | 26 | | 1.6 |
| | 100 | 182 | 72 | 28 | | 2.1 |
| Dodecane | 25 | 108 | | | | |
| | 50 | 127 | | | Not 2 Phase | No TDA in overhead |
| | 75 | 145 | | | | |
| Decaline | 25 | 84 | | | Not 2 Phase | No TDA in overhead |
| Tetradecene | 25 | 136 | 94 | 6 | | 12.1 |
| | 50 | 149 | 96 | 4 | | |
| | 75 | 165 | Single Phase | | 14 | 86 |
| Hexadecene | 25 | 139 | 82 | 18 | 49 | 0.3 |
| | 50 | 156 | 82 | 18 | 36 | 2.1 |
| | 75 | 189 | 82 | 18 | 31 | 2.6 |
| Xylene | - | TDA soluble in xylene - not evaluated further. | | | | |

The results show that tetradecane, tetradecene, hexadecane, or hexadecene were extremely effective in breaking the toluenediamine-N-tertiary-butyl-toltoluenediamine azeotrope. The upper layer in the decanter contains from about 5-30% toluenediamine. The percent hydrocarbon in the lower layer was typically less than 3%. Tetradecane is the preferred hydrocarbon. Decaline, dodecane, hexadecene and xylene were not effective since the solubility of TDA in the hydrocarbon was too great.

## Example 3

Approximately 2,220 pounds of crude material containing N-tertiary-butyl-toluenediamine and 127 pounds of tetradecane were charged to a batch still containing 8 inch by 24 feet of Koch-Sulzer packing. An overheads portion was removed and decanted with the top layer consisting largely of tetradecane being returned to the column as reflux. The bottoms from the decanter were removed as product and consisted largely of toluenediamine. When no separation was occurring at the decanter, the decanter was by-passed and overheads were removed at an 8 to 1 reflux ratio and tetradecane, N-tertiary-butyl-toluenediamine and tertiary-butyl-toluenediamine were recovered in that order.

The results showing pounds of product produced and composition are set forth in Table 2.

TABLE 2

| Material Recovered | |
|---|---|
| TDA | 709 lbs @ 100% |
| | 12 lbs @ 6.8% |
| Total | 721 lbs |
| NTBTDA | 377 lbs @ 94% |
| | 122 lbs @ 72% |
| | 19 lbs @ 18% |
| Total | 518 lbs |
| TBTDA | 14 lbs @ 8% |
| | 40 lbs @ 38% |
| | 320 lbs @ 79% |
| | 226 lbs % 75% |
| Total | 600 |
| DTBTDA & Others | 18 lbs @ 4.6% |
| | 37 lbs @ 36% |
| | 81 lbs @ 20% |
| | 249 lbs @ 24% |
| Total | 385 |
| TBTDA refers to mono-tert-butyl-toluenediamine and DTBTDA refers to ditertiary-butyl-toluenediamine. | |

The results in Table 2 show the recovery of the various materials and their concentration. Approximately 73% of the charged N-tertiary butyl toluenediamine was recovered at a purity of 94% with approximately 5% being N,N'-ditertiary-butyl-toluenediamine. It is believed the 27% remaining N-tertiary-butyl could easily be recovered by recycling the cuts to the next distillation. The azeotrope had been broken through the use of tetradecane.

## Claims

1. In a process for the separation of toluenediamine and N-tertiary-butyl-toluenediamine by distillation, the improvement for enhancing the breakup of an azeotrope formed during the process of separating N-tertiary-butyl-toluenediamine from toluenediamine which comprises contacting the azeotrope with a $C_{14-16}$ hydrocarbon capable of forming an azeotrope having a boiling point of at least 20°C lower than the boiling point of the toluenediamine-N-tertiary-butyltoluenediamine azeotrope and a solubility of toluenediamine in the hydrocarbon of nct greater than 30 grams per 100 grams of material.

2. The process of Claim 1 wherein the proportion of hydrocarbon to toluenediamine-N-tertiary-butyl-toluenediamine azeotrope as from 50 to 500 parts per 100 parts toluenediamine-N-tertiary-butyl-toluenediamine azeotrope.

3. The process of Claim 2 wherein said hydrocarbon is tetradecane, tetradecene, hexadecane.

4. The process of Claim 3 wherein said hydrocarbon is tetradecane.

5. The process of Claim 4 wherein said tetradecane is used in a proportion of from 75-150 parts/100 parts of azeotrope.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-1 555 382 (THE LUMMUS COMPANY)<br>* claim 1 * | 1 | C 07 C 85/26<br>C 07 C 87/62<br>C 07 C 87/58<br>B 01 D 3/36 |
| A,D | US-A-2 692 287 (ALAN BELL et al.)<br>* column 5, example 6; claim 1 * | 1 | |
| A,D | US-A-2 692 288 (ALAN BELL et al.)<br>* column 4, example 4; column 5, line 1<br>- 18; claim 1 * | 1 | |
| A | EP-A-0 231 496 (UOP INC)<br>* abstract * | | |
| A | US-A-4 276 126 (BERNARD A. SAFFER)<br>* abstract * | | |

TECHNICAL FIELDS
SEARCHED (Int. Cl.4)

C 07 C 85/00
C 07 C 87/00
B 01 D 3/00

The present search report has been drawn up for all claims

| Place of search | Date of completion of the search | Examiner |
|---|---|---|
| BERLIN | 29-01-1989 | RUFET J.M.A. |